# EUROPEAN PATENT APPLICATION

(11) **EP 4 850 680 A1**
(43) Date of publication of application: **09.12.2026**
(21) Application number: 25748715.7
(22) Date of filing: 30.01.2025
(51) Int. Cl.: A61K 38/16, A61K 9/10, A61P 1/04, A61P 9/00, A61P 9/08, A61P 9/12, A61P 25/28, A61P 31/00, A61P 37/02, C07K 14/575

(54) **ADRENOMEDULLIN ADMINISTRATION METHOD**

(30) Priority: 30.01.2024 JP 2024011993
(71) Applicant: National Cerebral and Cardiovascular Center, Suita-shi, Osaka 564-8565 (JP)
(72) Inventor: IHARA, Masafumi, Kishibe-Shimmachi, Suita-shi, Osaka 564-8565 (JP); SAITO, Satoshi, Kishibe-Shimmachi, Suita-shi, Osaka 564-8565 (JP); TANAKA, Tomotaka, Kishibe-Shimmachi, Suita-shi, Osaka 564-8565 (JP); YOSHIMOTO, Takeshi, Kishibe-Shimmachi, Suita-shi, Osaka 564-8565 (JP)
(74) Representative: Weickmann & Weickmann PartmbB
(86) International application number: PCT/JP2025/002967
(87) International publication number: WO 2025/164712

(57) **Abstract**

An administration method that maximizes the therapeutic effect of adrenomedullin is provided, and a medicament containing adrenomedullin that is applied by the administration method is provided. The above-mentioned problem is solved by providing a medicament for treating a disease, comprising, as an active ingredient, adrenomedullin or a derivative thereof having adrenomedullin activity, or the like, wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and wherein the aforementioned active ingredient is intermittently administered.

## Description

### [Technical Field]

The present invention relates to a method of administering adrenomedullin. The present invention also relates to a medicament containing adrenomedullin, which is applied by intermittent administration.

### [Background Art]

Adrenomedullin (hereinafter also referred to as "AM") is a physiologically active peptide isolated and identified from pheochromocyte tissue in 1993. At the time of its discovery, AM was found to exert a potent vasodilatory hypotensive action. Furthermore, subsequent studies revealed that AM exerts various pharmacological actions, such as an anti-inflammatory action, an angiogenic action, a cardiovascular protective action, a tissue repair-promoting action, and the like.

Since AM exerts the pharmacological actions as described above, it is expected to be useful as a therapeutic drug for cerebrovascular diseases, dementia, inflammatory bowel diseases, autoimmune diseases, infectious diseases, pulmonary hypertension, peripheral vascular diseases, heart diseases, and the like.

For example, Non Patent Literature 1 describes an implementation plan for the investigator-initiated clinical trial AMFIS trial for examining the safety and efficacy of AM in the treatment of ischemic stroke.

### [Citation List]

### [Non Patent Literature]

[Non Patent Literature 1]
Yoshimoto et al., Journal of Stroke and Cerebrovascular Diseases, vol. 30 (6), 105761, 2021

### [Summary of Invention]

### [Technical Problem]

However, up to now, an administration method that maximizes the therapeutic effect of AM has not been known.

Accordingly, an object of the present invention is to provide an administration method that maximizes the therapeutic effect of AM, and to provide a medicament comprising AM that is applied by the administration method.

### [Solution to Problem]

The present inventors have conducted intensive studies in an attempt to solve the above-mentioned problems and unexpectedly found for the first time that intermittent administration of AM has a high therapeutic effect in the investigator-initiated clinical trial AMFIS trial in which AM was administered to patients with acute cerebral infarction. Based on the finding, they have completed the present invention.

That is, one aspect of the present invention relates to the following.
[1] A medicament for treating a disease, comprising, as an active ingredient, adrenomedullin or a derivative thereof having adrenomedullin activity, or a pharmaceutically acceptable salt thereof, or a pharmaceutically acceptable solvate thereof,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   wherein the aforementioned active ingredient is intermittently administered.
[2] The medicament according to [1], wherein the aforementioned adrenomedullin or the derivative thereof having adrenomedullin activity is a peptide selected from the group consisting of the following:
   (i) a peptide consisting of the amino acid sequence of adrenomedullin,
   (ii) a peptide consisting of the amino acid sequence of adrenomedullin, wherein two cysteine residues in the amino acid sequence form a disulfide bond,
   (iii) the peptide of (ii), wherein the aforementioned disulfide bond is substituted by an ethylene group, and the peptide has adrenomedullin activity,
   (iv) the peptide of any of (i) to (iii), wherein 1 to 15 amino acid residues are deleted, substituted, or added, and the peptide has adrenomedullin activity,
   (v) the peptide of any of (i) to (iv), wherein the C-terminus is amidated, and
   (vi) the peptide of any of (i) to (iv), wherein a glycine residue is added to the C-terminus.
[3] The medicament according to [1] or [2], wherein the aforementioned adrenomedullin or the derivative thereof having adrenomedullin activity is a peptide selected from the group consisting of the following:
   (a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
   (b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
   (c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
   (d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
   (e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
   (f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
   (g) the peptide of any of (a) to (f), wherein the aforementioned disulfide bond is substituted by an ethylene group, and the peptide has adrenomedullin activity;
   (h) the peptide of any of (a) to (g), wherein 1 to 15 amino acid residues are deleted, substituted, or added, and the peptide has adrenomedullin activity;
   (i) the peptide of any of (a) to (h), wherein the C-terminus is amidated; and
   (j) the peptide of any of (a) to (h), wherein a glycine residue is added to the C-terminus.
[4] The medicament according to any of [1] to [3], wherein the aforementioned disease is a disease for which a therapeutic effect is expected by the anti-inflammatory action, angiogenic action, cardiovascular protective action and/or tissue repair-promoting action of adrenomedullin or the derivative thereof having adrenomedullin activity, or the pharmaceutically acceptable salt thereof, or the pharmaceutically acceptable solvate thereof.
[5] The medicament according to any of [1] to [4], wherein the aforementioned disease is at least one selected from the group consisting of cerebrovascular diseases, dementia, inflammatory bowel diseases, autoimmune diseases, infectious diseases, pulmonary hypertension, peripheral vascular diseases, and heart diseases.
[6] The medicament according to any of [1] to [5], wherein the intermittent administration is administration for not more than 23 hours per day.
[7] The medicament according to any of [1] to [6], wherein the intermittent administration is administration for not more than 23 hours per day, and is performed for 3 to 30 days.
[8] A method for treating a disease, comprising administering to a subject a therapeutically effective amount of adrenomedullin or a derivative thereof having adrenomedullin activity, or a pharmaceutically acceptable salt thereof, or a pharmaceutically acceptable solvate thereof,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   wherein the aforementioned administration is intermittent administration.
[9] Adrenomedullin or a derivative thereof having adrenomedullin activity, or a pharmaceutically acceptable salt thereof, or a pharmaceutically acceptable solvate thereof, for use in the treatment of a disease,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   which is intermittently administered.
[10] Use of adrenomedullin or a derivative thereof having adrenomedullin activity, or a pharmaceutically acceptable salt thereof, or a pharmaceutically acceptable solvate thereof, in the manufacture of a therapeutic medicament for a disease,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   wherein the aforementioned therapeutic medicament is intermittently administered.

### [Advantageous Effects of Invention]

According to the present invention, since an optimized administration method of AM is provided, the therapeutic effect of AM can be exerted to the maximum extent. In addition, by reducing the total administered amount of AM, it becomes possible to reduce side effects. Furthermore, according to the present invention, since a therapeutic effect can be obtained by intermittent administration, hospitalization is not necessarily required, and outpatient treatment becomes possible. Accordingly, not only does administration of AM at clinics and the like having no hospitalization facilities become possible, but also administration at home or at nursing care facilities and the like becomes possible.

### [Description of Embodiments]

One example of the embodiments of the present invention is described in detail in the following, but the present invention is not limited thereto.

### (Summary of the present invention)

As described above, AM is a substance that exerts various pharmacological actions, such as an anti-inflammatory action, an angiogenic action, a cardiovascular protective action, a tissue repair-promoting action, and the like. Since AM exerts such pharmacological actions, it is expected to be useful as a therapeutic drug for cerebrovascular diseases, dementia, inflammatory bowel diseases, autoimmune diseases, infectious diseases, pulmonary hypertension, peripheral vascular diseases, heart diseases, and the like.

Incidentally, since AM has a short half-life of 22 minutes in the body, the optimum administration method to exert a therapeutic effect has been considered to be continuous administration of a sufficient amount of AM over a long period of time. However, up to now, no detailed investigation has been conducted regarding an administration method for maximizing the therapeutic effect of AM.

The present inventors conducted intensive studies of the above-mentioned problem, and found that a high therapeutic effect can be obtained by intermittent administration, in an investigator-initiated clinical trial AMFIS trial in which AM was administered to patients with acute cerebral infarction. More specifically, the present inventors completely unexpectedly and accidentally found that a superior improvement effect on motor function can be obtained by intermittent administration in which AM is administered for 7 days at a pace of 8 hr/day from immediately after the onset of cerebral infarction. This result is a surprising result that could not even have been predicted from the conventional recognition that continuous administration over a long period of time is necessary in order to exert its effect, because of the short half-life of AM described above.

According to the present invention, an administration method can be provided which is superior to conventional administration methods in terms of exerting the effect of AM. In addition, according to the present invention, since a therapeutic effect can be obtained by intermittent administration, there is a great advantage in that hospitalization is not necessarily required and outpatient treatment becomes possible. Accordingly, the present invention is extremely promising in the treatment of various diseases for which a therapeutic effect is expected by administration of adrenomedullin or a derivative thereof having adrenomedullin activity, or a pharmaceutically acceptable salt thereof, or a pharmaceutically acceptable solvate thereof (hereinafter at times referred to as "AM, etc.").

### (Definition)

In the present specification, "intermittent administration" means that continuous intravenous administration of a therapeutic drug is performed for a certain period of time, and the therapeutic drug is not administered during the remaining period of time. Generally, intermittent administration is performed by setting, with one day as one unit, a period of continuous intravenous administration of the therapeutic drug for any period of time and a drug-free period for the remaining period of time, and repeating this.

In the present specification, "treatment" means obtaining a desired pharmacological and/or physiological effect. The effect may be prophylactic from the viewpoint of completely or partially preventing a disease or a symptom thereof, or therapeutic from the viewpoint of partially or completely curing a disease and/or a side effect caused by the disease. That is, in the present specification, "prevention" is also included in the concept of "treatment".

"Treatment" refers to any treatment of a disease in mammals, particularly humans, including, for example, the following: (a) preventing the occurrence of a disease in a subject who may be predisposed to the disease but has not yet been diagnosed as having the disease; (b) suppressing the disease, i.e., preventing the development of the disease; and (c) alleviating the disease (i.e., causing regression of the disease). When "treatment" is the above-described therapeutic treatment, it may be, for example, suppressing (for example, suppressing progression of), ameliorating, repairing, and/or curing symptoms that have occurred (onset or manifestation) in a subject suffering from the disease.

In the present specification, "therapeutically effective amount" means an amount of an active ingredient sufficient to effectively perform such treatment of a disease when administered to a subject. The "therapeutically effective amount" varies depending on the active ingredient, the disease and the severity thereof, and the age, body weight, and the like of the subject to be treated.

In the present specification, "subject" means a test subject or a patient requiring treatment by administration of AM, etc.

In the present specification, "acute cerebral infarction" generally means cerebral infarction within 72 hours after onset, particularly within 24 hours after onset. Cerebral infarction is classified, based on the mechanism of onset, into embolic cerebral infarction and non-embolic (for example, thrombotic or hemodynamic) cerebral infarction, and is also classified, based on clinical subtype, into lacunar infarction, atherothrombotic cerebral infarction, cardiogenic cerebral embolism, and the like.

### (Adrenomedullin)

In each aspect of the present invention, AM may be not only a human-derived peptide (SEQ ID NO: 1) isolated and identified from human pheochromocyte tissue, but also, for example, a peptide (ortholog) derived from another non-human mammal (for example, a warm-blooded animal), such as pig (SEQ ID NO: 4), dog (SEQ ID NO: 6), bovine (SEQ ID NO: 8), rat (SEQ ID NO: 10), or mouse (SEQ ID NO: 12). In vivo, these peptides have a disulfide bond formed by two cysteine residues in the amino acid sequence thereof, and the C-terminus is amidated. In the present specification, the aforementioned peptide having the disulfide bond and the C-terminal amide group is sometimes referred to as "natural adrenomedullin" or simply "adrenomedullin." In each aspect of the present invention, any of the aforementioned peptides can be applied as the active ingredient.

In the present specification, "C-terminal amidation" means one form of post-translational modification of a peptide in vivo, and specifically means a reaction in which the main-chain carboxyl group of the C-terminal amino acid residue of the peptide is converted into an amide group. Further, in the present specification, "formation of a disulfide bond between cysteine residues" or "disulfidation of cysteine residues" means one form of post-translational modification of a peptide in vivo, and specifically means a reaction in which two cysteine residues in the amino acid sequence of the peptide form a disulfide bond (-S-S-). Many physiologically active peptides produced in vivo are first biosynthesized as precursor proteins having a larger molecular weight, and during the process of intracellular transport, these undergo post-translational modification reactions such as C-terminal amidation and/or disulfidation of cysteine residues to become mature physiologically active peptides. C-terminal amidation generally proceeds by the action of a C-terminal amidating enzyme on the precursor protein. In the case of a physiologically active peptide having a C-terminal amide group, in the precursor protein thereof, a Gly residue is bonded to the C-terminal carboxyl group to be amidated, and the Gly residue is converted into the C-terminal amide group by the C-terminal amidating enzyme. Further, in the C-terminal propeptide of the precursor protein, there is present a repetitive sequence of a combination of basic amino acid residues such as Lys-Arg or Arg-Arg (Mizuno, Seikagaku, Vol. 61, No. 12, pp. 1435-1461 (1989)). Disulfidation of cysteine residues can proceed under oxidative conditions. In vivo, disulfidation of cysteine residues generally proceeds by the action of protein disulfide isomerase on the precursor protein.

### (Adrenomedullin derivative)

In each aspect of the present invention, not only natural-type AM itself but also derivatives thereof having adrenomedullin activity may be used as the active ingredient. In each aspect of the present invention, the "derivative of adrenomedullin" or "adrenomedullin derivative" means a compound having, as a partial structure thereof, a peptide chain corresponding to AM. Examples of the AM derivative having adrenomedullin activity include, but are not limited to, the compounds disclosed in the specifications of WO 2015/141819, WO 2017/047788, and WO 2018/181638. A person skilled in the art can prepare the compound by, based on the aforementioned documents, purchasing the AM derivative having adrenomedullin activity, or applying an appropriate conversion reaction to the purchased compound, or preparing the compound by himself/herself. The AM derivatives disclosed in the aforementioned documents can continuously exhibit the pharmacological effects of adrenomedullin without substantially causing undesirable side effects. Therefore, in each aspect of the present invention, by using the AM derivative disclosed in the aforementioned documents as the active ingredient, a disease for which a therapeutic effect is expected by administration of AM, etc. can be treated through the adrenomedullin activity possessed by the AM derivative, while substantially avoiding the occurrence of undesirable side effects.

In each aspect of the present invention, "adrenomedullin activity" means, for example, various physiological actions exemplified below, and particularly means physiological actions that may be involved in the treatment of symptoms of cerebral infarction, such as an action for controlling inflammation of cerebral infarction through the anti-inflammatory action possessed by AM, an action for alleviating tissue injury due to acute ischemia through the vasodilatory action possessed by AM, and an action for inducing vascular regeneration through the angiogenic action possessed by AM.
(1) Cardiovascular system: vasodilatory action, blood pressure-lowering action, blood pressure elevation-suppressing action, cardiac output-increasing/heart failure-improving action, pulmonary hypertension-improving action, angiogenic action, lymphangiogenic action, vascular endothelial function-improving action, anti-arteriosclerotic action, cardioprotective action (for example, cardioprotective action in ischemia-reperfusion injury or inflammation), post-myocardial infarction remodeling-suppressing action, cardiac hypertrophy-suppressing action, and angiotensin-converting enzyme inhibitory action.
(2) Kidney/water-electrolyte system: diuretic action, natriuretic action, antidiuretic hormone-suppressing action, aldosterone-lowering action, renoprotective action (for example, renoprotective action in hypertension or ischemia-reperfusion injury), drinking behavior-suppressing action, and salt appetite-suppressing action.
(3) Brain/nervous system: neuroprotective/cerebral injury-suppressing action, anti-inflammatory action (for example, an action for controlling inflammation of cerebral infarction), vasodilatory action (for example, an action for alleviating tissue injury due to acute ischemia), angiogenic action (for example, an action for inducing vascular regeneration), apoptosis-suppressing action (for example, an apoptosis-suppressing action in ischemia-reperfusion injury or inflammation), autoregulatory function-maintaining action, oxidative stress-suppressing action, dementia-improving action, and sympathetic nerve-suppressing action.
(4) Urogenital system: erection-improving action, blood flow-improving action, and implantation-promoting action.
(5) Digestive system: anti-ulcer action, tissue repair action, mucosal regeneration action, blood flow-improving action, anti-inflammatory action, and liver function-improving action.
(6) Orthopedic system: osteoblast-stimulating action and arthritis-improving action.
(7) Endocrine/metabolic system: adipocyte differentiation action, lipolysis-regulating action, insulin sensitivity-improving action, insulin secretion-regulating action, antidiuretic hormone secretion-suppressing action, and aldosterone secretion-suppressing action.
(8) Others: circulation-improving action, anti-inflammatory action, cytokine-regulating action, organ-protective action, oxidative stress-suppressing action, tissue repair action (for example, anti-pressure ulcer action), septic shock-improving action, multiple organ failure-suppressing action, autoimmune disease-suppressing action, antibacterial action, hair growth-promoting action, and hair nourishing action.

The aforementioned adrenomedullin or a derivative thereof having adrenomedullin activity is preferably a peptide selected from the group consisting of the following:
(i) a peptide consisting of the amino acid sequence of adrenomedullin,
(ii) a peptide consisting of the amino acid sequence of adrenomedullin, wherein two cysteine residues in the amino acid sequence form a disulfide bond,
(iii) the peptide of (ii), wherein the aforementioned disulfide bond is substituted by an ethylene group, and the peptide has adrenomedullin activity,
(iv) the peptide of any of (i) to (iii), wherein 1 to 15 amino acids are deleted, substituted, or added, and the peptide has adrenomedullin activity,
(v) the peptide of any of (i) to (iv), wherein the C-terminus is amidated, and
(vi) the peptide of any of (i) to (iv), wherein a glycine residue is added to the C-terminus.

In one embodiment of the present invention, the aforementioned adrenomedullin or a derivative thereof having adrenomedullin activity is more preferably a peptide selected from the group consisting of the following:
(i) a peptide consisting of the amino acid sequence of adrenomedullin,
(ii) a peptide consisting of the amino acid sequence of adrenomedullin, wherein two cysteine residues in the amino acid sequence form a disulfide bond,
(v) the peptide of (i) or (ii), wherein the C-terminus is amidated, and
(vi) the peptide of (i) or (ii), wherein a glycine residue is added to the C-terminus.

Among the aforementioned peptides of (i) to (vi), the peptide included in (v), consisting of the amino acid sequence of adrenomedullin, wherein the C-terminus is amidated, and wherein two cysteine residues in the amino acid sequence form a disulfide bond, corresponds to mature natural-type adrenomedullin. The peptide of (i) consisting of the amino acid sequence of adrenomedullin corresponds to natural-type adrenomedullin in the form before undergoing post-translational modifications of C-terminal amidation and disulfidation of cysteine residues (that is, the immature form). Among the aforementioned peptides of (i) to (vi), peptides other than the peptide described above correspond to derivatives (modified forms) of adrenomedullin.

The aforementioned peptide of (ii) can be formed by subjecting the thiol groups of the two cysteine residues of the aforementioned peptide of (i) to air oxidation, or by oxidizing the thiol groups using an appropriate oxidizing agent and converting them into a disulfide bond. By using the aforementioned peptide of (ii), the three-dimensional structure of the peptide can be made similar to the three-dimensional structure of natural-type adrenomedullin. As a result, the adrenomedullin activity of the aforementioned peptide of (ii) can be made substantially equivalent to that of natural-type adrenomedullin.

The aforementioned peptide of (iii) can be formed by converting the disulfide bond of the aforementioned peptide of (ii) into an ethylene group. The substitution from the disulfide bond to the ethylene group can be performed by a method well known in the art (O. Keller et al., Helv. Chim. Acta, 1974, Vol. 57, p. 1253). By using the aforementioned peptide of (iii), the three-dimensional structure of the peptide can be stabilized. As a result, the aforementioned peptide of (iii) can continuously exhibit adrenomedullin activity in vivo.

In the aforementioned peptide of (iv), the number of amino acid residues deleted, substituted, or added is preferably in the range of 1 to 15, more preferably in the range of 1 to 10, further preferably in the range of 1 to 8, particularly preferably in the range of 1 to 5, and most preferably in the range of 1 to 3. A preferred peptide of (iv) is a peptide of any of the peptides (i) to (iii), wherein amino acid residues at positions 1 to 15, 1 to 12, 1 to 10, 1 to 8, 1 to 5, or 1 to 3 from the N-terminal side are deleted, and which has adrenomedullin activity, and a more preferred peptide of (iv) is a peptide of any of the peptides (i) to (iii), wherein amino acid residues at positions 1 to 15, 1 to 10, or 1 to 5 from the N-terminal side are deleted, and which has adrenomedullin activity. In the aforementioned preferred peptide, one or more (for example, 1 to 5, 1 to 3, or 1 or 2) amino acid residues may be further deleted, substituted, or added. By using the aforementioned peptide of (iv), the adrenomedullin activity of the peptide can be made substantially equivalent to that of natural-type adrenomedullin. Further, by using the aforementioned peptide of (iv), the peptide can continuously exhibit adrenomedullin activity in vivo.

The aforementioned peptide of (vi) can be converted into the aforementioned peptide of (v) by conversion of the glycine residue at the C-terminus into a C-terminal amide group through the action of a C-terminal amidating enzyme. Therefore, by administering the aforementioned peptide of (vi) to a subject, a C-terminal amidated peptide can be formed in the body of the subject after a certain period of time has elapsed. As a result, the aforementioned peptide of (vi) can continuously exhibit adrenomedullin activity in vivo.

The aforementioned adrenomedullin or a derivative thereof having adrenomedullin activity is more preferably a peptide selected from the group consisting of the following:
(a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
(b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
(c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
(d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
(e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
(f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
(g) the peptide of any of (a) to (f), wherein the aforementioned disulfide bond is substituted by an ethylene group, and the peptide has adrenomedullin activity;
(h) the peptide of any of (a) to (g), wherein 1 to 15 amino acids are deleted, substituted, or added, and the peptide has adrenomedullin activity;
(i) the peptide of any of (a) to (h), wherein the C-terminus is amidated; and
(j) the peptide of any of (a) to (h), wherein a glycine residue is added to the C-terminus.

In one embodiment of the present invention, the aforementioned adrenomedullin or a derivative thereof having adrenomedullin activity is further preferably a peptide selected from the group consisting of the following:
(a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
(b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
(c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
(d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
(e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
(f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
(i) the peptide of any of (a) to (f), wherein the C-terminus is amidated; and
(j) the peptide of any of (a) to (f), wherein a glycine residue is added to the C-terminus.

In the aforementioned peptide of (h), the number of amino acid residues deleted, substituted, or added is preferably in the range of 1 to 12, more preferably in the range of 1 to 10, further preferably in the range of 1 to 8, particularly preferably in the range of 1 to 5, and most preferably in the range of 1 to 3. A preferred peptide of (h) is a peptide of any of the peptides (a) to (g), wherein amino acids at positions 1 to 15, 1 to 12, 1 to 10, 1 to 8, 1 to 5, or 1 to 3 from the N-terminal side are deleted, and which has adrenomedullin activity, and a more preferred peptide of (h) is a peptide of any of the peptides (a) to (d), wherein amino acid residues at positions 1 to 15, 1 to 10, or 1 to 5 from the N-terminal side are deleted, and which has adrenomedullin activity, or a peptide of (e) or (f), wherein amino acid residues at positions 1 to 13, 1 to 8, or 1 to 5 from the N-terminal side are deleted, and which has adrenomedullin activity. In the aforementioned preferred peptide, one or more (for example, 1 to 5, 1 to 3, or 1 or 2) amino acids may be further deleted, substituted, or added. By using the aforementioned peptide of (h), the adrenomedullin activity of the peptide can be made substantially equivalent to that of natural-type adrenomedullin. Further, by using the aforementioned peptide of (h), the peptide can continuously exhibit adrenomedullin activity in vivo.

### (Salts of adrenomedullin or derivatives thereof)

In each aspect of the present invention, the AM or a derivative thereof having adrenomedullin activity used as the active ingredient includes not only the compound itself but also salts thereof. When the AM or a derivative thereof having adrenomedullin activity is in the form of a salt, the salt is preferably a pharmaceutically acceptable salt. Examples of the counterion of the salt of AM or a derivative thereof having adrenomedullin activity include, but are not limited to, cations such as sodium ion, potassium ion, calcium ion, magnesium ion, or substituted or unsubstituted ammonium ion; or anions such as chloride ion, bromide ion, iodide ion, phosphate ion, nitrate ion, sulfate ion, carbonate ion, hydrogen carbonate ion, perchlorate ion, formate ion, acetate ion, trifluoroacetate ion, propionate ion, lactate ion, maleate ion, hydroxymaleate ion, methylmaleate ion, fumarate ion, adipate ion, benzoate ion, 2-acetoxybenzoate ion, *p*-aminobenzoate ion, nicotinate ion, cinnamate ion, ascorbate ion, pamoate ion, succinate ion, salicylate ion, bismethylenesalicylate ion, oxalate ion, tartrate ion, malate ion, citrate ion, gluconate ion, aspartate ion, stearate ion, palmitate ion, itaconate ion, glycolate ion, glutamate ion, benzenesulfonate ion, cyclohexylsulfamate ion, methanesulfonate ion, ethanesulfonate ion, isethionate ion, benzenesulfonate ion, *p*-toluenesulfonate ion, or naphthalenesulfonate ion. When the AM or a derivative thereof having adrenomedullin activity is in the form of a salt with the aforementioned counterion, the adrenomedullin activity of the compound can be made substantially equivalent to that of natural-type adrenomedullin.

### (Solvates of adrenomedullin or salts thereof)

In each aspect of the present invention, the AM or a derivative thereof having adrenomedullin activity used as the active ingredient includes not only the compound itself but also solvates of the compound or salts thereof. When the AM or a derivative thereof having adrenomedullin activity, or a salt thereof, is in the form of a solvate, the solvate is preferably a pharmaceutically acceptable solvate. Examples of the solvent capable of forming a solvate with the compound or a salt thereof include, but are not limited to, water, or an organic solvent such as methanol, ethanol, 2-propanol (isopropyl alcohol), dimethyl sulfoxide (DMSO), acetic acid, ethanolamine, acetonitrile, or ethyl acetate. When the AM or a derivative thereof having adrenomedullin activity, or a salt thereof, is in the form of a solvate with the above solvent, the adrenomedullin activity of the compound can be made substantially equivalent to that of natural-type adrenomedullin.

In addition, in each aspect of the present invention, the AM or a derivative thereof having adrenomedullin activity used as the active ingredient includes not only the compound itself but also individual enantiomers and diastereomers of the compound, as well as mixtures of stereoisomers of the compound, such as racemates.

### (Disease)

In each aspect of the present invention, the disease is a disease for which a therapeutic effect is expected by administration of AM. Since the present invention provides an administration method for maximizing the therapeutic effect of AM, the present invention is applicable to all diseases treated by administration of AM. That is, the disease for which a therapeutic effect is expected by administration of AM may be any disease treated by administration of AM. In other words, such a disease is a disease for which a therapeutic effect is expected (treated) by the anti-inflammatory action, angiogenic action, cardiovascular protective action, and/or tissue repair-promoting action of AM.

Examples of the disease for which a therapeutic effect is expected by administration of AM include cerebrovascular diseases, dementia, inflammatory bowel diseases, autoimmune diseases, infectious diseases, pulmonary hypertension, peripheral vascular diseases, heart diseases, and the like.

Examples of the cerebrovascular disease include stroke, cerebral infarction (for example, cerebral thrombosis, cerebral embolism, and the like), cerebral hemorrhage (for example, intracerebral hemorrhage, subarachnoid hemorrhage, and the like), acute epidural hematoma, acute subdural hematoma, chronic subdural hematoma, and the like.

Examples of the dementia include Alzheimer's disease (AD), vascular dementia, dementia with Lewy bodies, Pick's disease, frontotemporal dementia (FTD), dementia due to AIDS, age-related cognitive impairment, age-related memory impairment, and other neurodegenerative diseases associated with dementia.

Examples of the inflammatory bowel disease include ulcerative colitis, Crohn's disease, intestinal Behçet's disease, infectious enteritis, drug-induced enteritis, ischemic enteritis, intestinal tuberculosis, and the like.

Examples of the autoimmune disease include chronic rheumatoid arthritis, collagen diseases (for example, systemic lupus erythematosus and the like), multiple sclerosis, neuromyelitis optica, and the like.

Examples of the infectious disease include respiratory infections (for example, sepsis, pneumonia, and the like), urinary tract infections (for example, pyelonephritis and the like), gastrointestinal infections (for example, peritonitis, cholecystitis, and the like), burns, infections occurring after surgery, viral infections (for example, COVID-19 and the like), and the like. In one embodiment of the present invention, the infectious disease may be a severe infectious disease.

Examples of the pulmonary hypertension include pulmonary arterial hypertension, pulmonary hypertension associated with left heart disease, pulmonary hypertension due to lung disease or hypoxemia, chronic thromboembolic pulmonary hypertension, pulmonary hypertension due to unknown multifactorial causes, and the like.

Examples of the peripheral vascular disease include carotid artery stenosis, subclavian artery stenosis, renal artery stenosis, arteriosclerosis obliterans, acute arterial occlusion, Buerger's disease, varicose veins of the lower extremities, and the like.

Examples of the heart disease include myocardial infarction (for example, acute myocardial infarction, subacute myocardial infarction, old myocardial infarction, and the like), angina pectoris, heart failure, valvular disease, and the like. From the viewpoint of therapeutic effect, acute myocardial infarction is preferred.

### (Medicament)

In the medicament of the present aspect, the AM or a derivative thereof having adrenomedullin activity used as the active ingredient may be used alone, or may be used in combination with one or more pharmaceutically acceptable components. The medicament of the present aspect may also be provided in the form of a pharmaceutical composition containing adrenomedullin or a derivative thereof having adrenomedullin activity and one or more pharmaceutically acceptable carriers. In this embodiment, the pharmaceutical composition may further contain, in addition to the aforementioned components, one or more pharmaceutically acceptable additives such as media (for example, a solvent such as sterile water or a solution such as physiological saline), excipients, binders, vehicles, solubilizing agents, preservatives, stabilizers, disintegrants, disintegration inhibitors, swelling agents, lubricants, surfactants, emulsifiers, oily liquids (for example, vegetable oils), suspending agents, buffering agents, pain-relieving agents, antioxidants, sweetening agents, and flavoring agents.

The dosage form of the medicament of the present aspect is not particularly limited as long as it is a preparation for use in intravenous administration. Examples of the preparation for use in intravenous administration include injections such as sterile solutions or suspensions with water or another pharmaceutically acceptable liquid. Examples of additives that can be mixed into the injection include, but are not limited to, vehicles such as isotonic solutions containing physiological saline, glucose, or other auxiliary agents (for example, D-sorbitol, D-mannitol, or sodium chloride), solubilizing agents such as alcohols (for example, ethanol or benzyl alcohol), esters (for example, benzyl benzoate), or polyalcohols (for example, propylene glycol or polyethylene glycol), nonionic surfactants such as polysorbate 80 or polyoxyethylene hydrogenated castor oil, oily liquids such as sesame oil or soybean oil, buffering agents such as phosphate buffer solution or sodium acetate buffer solution, pain-relieving agents such as benzalkonium chloride or procaine hydrochloride, stabilizers such as human serum albumin or polyethylene glycol, preservatives, antioxidants, and the like. The prepared injection is generally filled into an appropriate container (for example, a vial or ampoule) and stored under an appropriate environment until use.

The medicament of the present aspect may also be used in combination with one or more other agents useful as pharmaceuticals. In this case, the medicament of the present aspect may be provided in the form of a single pharmaceutical comprising AM or a derivative thereof having adrenomedullin activity and one or more other agents, or may be provided in the form of a pharmaceutical combination or kit comprising a plurality of formulations in which AM or a derivative thereof having adrenomedullin activity and one or more other agents are formulated separately. In the case of a pharmaceutical combination or kit, the respective formulations may be administered simultaneously or separately (for example, sequentially).

When AM or a derivative thereof having adrenomedullin activity is applied to a pharmaceutical use, the AM or a derivative thereof having adrenomedullin activity includes not only the compound itself but also pharmaceutically acceptable salts of the compound and pharmaceutically acceptable solvates thereof. Examples of the pharmaceutically acceptable salts of AM or a derivative thereof having adrenomedullin activity, and the pharmaceutically acceptable solvates thereof include, but are not limited to, the salts or solvates exemplified above. When the AM or a derivative thereof having adrenomedullin activity is in the form of the aforementioned salt or solvate, the compound can be applied to a desired pharmaceutical use.

The AM or a derivative thereof having adrenomedullin activity used as the active ingredient of the medicament of the present aspect is derived from adrenomedullin, which is a natural physiologically active peptide. Therefore, the AM or a derivative thereof having adrenomedullin activity is safe and has low toxicity. Accordingly, the medicament of the present aspect can be applied to various subjects requiring treatment of a disease for which a therapeutic effect is expected by administration of AM, etc. The aforementioned subject is preferably a test subject or patient of a human or a non-human mammal (for example, a warm-blooded animal such as a pig, dog, cow, rat, mouse, guinea pig, rabbit, chicken, sheep, cat, monkey, baboon, or chimpanzee), and more preferably a human patient. By intermittently administering the medicament of the present aspect to the aforementioned subject, the disease in the subject can be treated.

When the medicament of the present embodiment is administered to a subject, particularly a human patient, the attending physician should finally determine the therapeutically effective administration method and dose in consideration of many factors such as the age and sex of the subject, the exact condition (for example, severity) of the symptoms, disease and/or disorder to be prevented or treated, and the administration route. Therefore, in the medicament of the present aspect, the AM or a derivative thereof having adrenomedullin activity, which is the active ingredient, is administered to the subject by a therapeutically effective administration method and dose (for example, administration amount and administration route).

In the medicament of the present aspect, the AM or a derivative thereof having adrenomedullin activity, which is the active ingredient, is preferably administered intravenously intermittently. In this embodiment, the dosage form of the medicament of the present embodiment is, for example, an injection preparation such as a sterile solution or suspension with water or another pharmaceutically acceptable liquid (for example, physiological saline). The aforementioned intravenous administration is preferably performed at a rate in the range of 1.0 to 20.0 ng active ingredient/kg body weight/min, more preferably performed at a rate in the range of 6.0 to 12.0 ng active ingredient/kg body weight/min, in terms of AM. When the administration rate of the AM or a derivative thereof having adrenomedullin activity exceeds the above upper limit, undesirable side effects such as decrease or fluctuation in blood pressure, aggravation of cerebral infarction, or concomitant hemorrhagic cerebral infarction may be caused. In addition, when the administration rate of the AM or a derivative thereof having adrenomedullin activity is less than the aforementioned lower limit, a sufficient therapeutic effect may not be obtained. Therefore, by intermittently intravenously administering the medicament of the present aspect at an administration rate within the aforementioned range, the disease can be treated without causing undesirable side effects.

In the medicament of the present aspect, the intermittent administration is not limited to the cerebrovascular disease described in the Examples, and can likewise be applied to any disease for which a therapeutic effect is expected by administration of AM (for example, cerebrovascular diseases, dementia, inflammatory bowel diseases, autoimmune diseases, infectious diseases, pulmonary hypertension, peripheral vascular diseases, heart diseases, and the like). That is, in the Examples of the present application, a cerebrovascular disease was employed as one example of a disease for which intermittent administration of AM according to the present invention is performed, and the effect was confirmed.

In the medicament of the present aspect, the aforementioned intravenous administration is preferably performed intermittently for within 23 hr per day, more preferably performed intermittently for within 15 hr, still more preferably performed intermittently for within 12 hr, particularly preferably performed intermittently for within 10 hr, and especially preferably performed intermittently for within 8 hr per day. For example, after continuous intravenous administration of AM is performed for within 8 hr, a drug-free period of 16 hr or more is provided until the next start of administration, and thereafter this is repeated. Further, the aforementioned intravenous administration is preferably performed for 3 to 30 days under the aforementioned conditions (that is, intermittent administration for within 23 hr, within 15 hr, within 12 hr, within 10 hr, or within 8 hr per day), more preferably performed for 4 to 25 days, still more preferably performed for 5 to 20 days, particularly preferably performed for 6 to 16 days, especially preferably performed for 7 to 14 days, and most especially preferably performed for 7 days. When the administration period of the AM or a derivative thereof having adrenomedullin activity is less than the aforementioned lower limit, a sufficient therapeutic effect may not be obtained. Therefore, by intermittently intravenously administering the medicament of the present aspect for an administration period within the aforementioned range, the disease can be treated with a higher therapeutic effect.

In the medicament of the present aspect, the intermittent administration of the AM or a derivative thereof having adrenomedullin activity is preferably started immediately after onset of the disease (for example, cerebral infarction). In conventional administration methods, it has generally been considered effective, for example, to perform continuous administration for several days immediately after onset of the disease (for example, cerebral infarction), and then shift to intermittent administration. However, according to the present invention, the therapeutic effect can be enhanced by performing intermittent administration immediately after onset.

In the medicament of the present aspect, it is preferable that the AM or a derivative thereof having adrenomedullin activity, which is the active ingredient, is intravenously administered preferably at a rate in the range of 1.0 to 20.0 ng active ingredient/kg body weight/min, more preferably at a rate in the range of 6.0 to 12.0 ng active ingredient/kg body weight/min, and that the intravenous administration is performed based on an administration schedule in which continuous administration for 8 hr and no administration for 16 hr are repeated over 7 days after the start of administration. By intermittently intravenously administering the medicament of the present aspect based on the aforementioned administration schedule, the disease can be treated with a higher therapeutic effect.

In each aspect of the present invention, when the subject to which treatment by administration of the AM or a derivative thereof having adrenomedullin activity is applied is, for example, a subject having acute cerebral infarction, the therapeutic effect can be determined by, for example, administering a medicament or the like containing the AM or a derivative thereof having adrenomedullin activity as an active ingredient to the subject having acute cerebral infarction, and evaluating the symptoms of cerebral infarction in the subject using, for example, the stroke severity scale (SS), the modified Rankin Scale (mRS), clinical examinations (for example, hematological examinations and blood biochemical examinations), vital signs (for example, blood pressure and electrocardiogram), cranial imaging findings (for example, evaluation of the presence or absence of hemorrhage, the presence or absence of infarction, and the presence or absence of severe stenosis by CT and/or MR examination), nuclear medicine examination, or ultrasonography. For example, when the symptoms of cerebral infarction in a subject having acute cerebral infarction are evaluated using NIHSS, the change in the NIHSS after one week is, as an average value, for example, in the range of -1.5 to -4.5.

### (Other embodiments)

The present invention also relates to the following.
[8] A method for treating a disease, comprising administering to a subject a therapeutically effective amount of adrenomedullin or a derivative thereof having adrenomedullin activity, or a pharmaceutically acceptable salt thereof, or a pharmaceutically acceptable solvate thereof,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   wherein the aforementioned administration is intermittent administration.
[8a] The method according to [8], wherein the aforementioned adrenomedullin or the derivative thereof having adrenomedullin activity is a peptide selected from the group consisting of the following:
   (i) a peptide consisting of the amino acid sequence of adrenomedullin,
   (ii) a peptide consisting of the amino acid sequence of adrenomedullin, wherein two cysteine residues in the amino acid sequence form a disulfide bond,
   (iii) the peptide of (ii), wherein the aforementioned disulfide bond is substituted by an ethylene group, and the peptide has adrenomedullin activity,
   (iv) the peptide of any of (i) to (iii), wherein 1 to 15 amino acid residues are deleted, substituted, or added, and the peptide has adrenomedullin activity,
   (v) the peptide of any of (i) to (iv), wherein the C-terminus is amidated, and
   (vi) the peptide of any of (i) to (iv), wherein a glycine residue is added to the C-terminus.
[8b] The method according to [8] or [8a], wherein the aforementioned adrenomedullin or the derivative thereof having adrenomedullin activity is a peptide selected from the group consisting of the following:
   (a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
   (b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
   (c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
   (d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
   (e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
   (f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
   (g) the peptide of any of (a) to (f), wherein the aforementioned disulfide bond is substituted by an ethylene group, and the peptide has adrenomedullin activity;
   (h) the peptide of any of (a) to (g), wherein 1 to 15 amino acid residues are deleted, substituted, or added, and the peptide has adrenomedullin activity;
   (i) the peptide of any of (a) to (h), wherein the C-terminus is amidated; and
   (j) the peptide of any of (a) to (h), wherein a glycine residue is added to the C-terminus.
[8c] The method according to any of [8] to [8b], wherein the aforementioned disease is a disease for which a therapeutic effect is expected by the anti-inflammatory action, angiogenic action, cardiovascular protective action and/or tissue repair-promoting action of adrenomedullin or the derivative thereof having adrenomedullin activity, or the pharmaceutically acceptable salt thereof, or the pharmaceutically acceptable solvate thereof.
[8d] The method according to any of [8] to [8c], wherein the aforementioned disease is at least one selected from the group consisting of cerebrovascular diseases, dementia, inflammatory bowel diseases, autoimmune diseases, infectious diseases, pulmonary hypertension, peripheral vascular diseases, and heart diseases.
[8e] The method according to any of [8] to [8d], wherein the intermittent administration is administration for not more than 23 hours per day.
[8f] The method according to any of [8] to [8e], wherein the intermittent administration is administration for not more than 23 hours per day, and is performed for 3 to 30 days.

AM or a derivative thereof having adrenomedullin activity, or a pharmaceutically acceptable salt thereof, or a pharmaceutically acceptable solvate thereof, which is administered in the method of the present aspect, has characteristics similar to those of the active ingredient of the medicament of the present aspect described above. In addition, the method of the present aspect can be carried out by usage and dosage similar to those of the medicament of the present aspect described above. Furthermore, in the method of the present aspect, diseases similar to those for the medicament of the present aspect described above can be treated.

The present invention also relates to the following.
[9] Adrenomedullin or a derivative thereof having adrenomedullin activity, or a pharmaceutically acceptable salt thereof, or a pharmaceutically acceptable solvate thereof, for use in the treatment of a disease,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   which is intermittently administered.
[9a] The adrenomedullin or a derivative thereof having adrenomedullin activity, or a pharmaceutically acceptable salt thereof, or a pharmaceutically acceptable solvate thereof according to [9], wherein the aforementioned adrenomedullin or the derivative thereof having adrenomedullin activity is a peptide selected from the group consisting of the following:
   (i) a peptide consisting of the amino acid sequence of adrenomedullin,
   (ii) a peptide consisting of the amino acid sequence of adrenomedullin, wherein two cysteine residues in the amino acid sequence form a disulfide bond,
   (iii) the peptide of (ii), wherein the aforementioned disulfide bond is substituted by an ethylene group, and the peptide has adrenomedullin activity,
   (iv) the peptide of any of (i) to (iii), wherein 1 to 15 amino acid residues are deleted, substituted, or added, and the peptide has adrenomedullin activity,
   (v) the peptide of any of (i) to (iv), wherein the C-terminus is amidated, and
   (vi) the peptide of any of (i) to (iv), wherein a glycine residue is added to the C-terminus.
[9b] The adrenomedullin or a derivative thereof having adrenomedullin activity, or a pharmaceutically acceptable salt thereof, or a pharmaceutically acceptable solvate thereof according to [9] or [9a], wherein the aforementioned adrenomedullin or the derivative thereof having adrenomedullin activity is a peptide selected from the group consisting of the following:
   (a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
   (b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
   (c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
   (d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
   (e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
   (f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
   (g) the peptide of any of (a) to (f), wherein the aforementioned disulfide bond is substituted by an ethylene group, and the peptide has adrenomedullin activity;
   (h) the peptide of any of (a) to (g), wherein 1 to 15 amino acid residues are deleted, substituted, or added, and the peptide has adrenomedullin activity;
   (i) the peptide of any of (a) to (h), wherein the C-terminus is amidated; and
   (j) the peptide of any of (a) to (h), wherein a glycine residue is added to the C-terminus.
[9c] The adrenomedullin or a derivative thereof having adrenomedullin activity, or a pharmaceutically acceptable salt thereof, or a pharmaceutically acceptable solvate thereof according to any of [9] to [9b], wherein the aforementioned disease is a disease for which a therapeutic effect is expected by the anti-inflammatory action, angiogenic action, cardiovascular protective action and/or tissue repair-promoting action of adrenomedullin or the derivative thereof having adrenomedullin activity, or the pharmaceutically acceptable salt thereof, or the pharmaceutically acceptable solvate thereof.
[9d] The adrenomedullin or a derivative thereof having adrenomedullin activity, or a pharmaceutically acceptable salt thereof, or a pharmaceutically acceptable solvate thereof according to any of [9] to [9c], wherein the aforementioned disease is at least one selected from the group consisting of cerebrovascular diseases, dementia, inflammatory bowel diseases, autoimmune diseases, infectious diseases, pulmonary hypertension, peripheral vascular diseases, and heart diseases.
[9e] The adrenomedullin or a derivative thereof having adrenomedullin activity, or a pharmaceutically acceptable salt thereof, or a pharmaceutically acceptable solvate thereof according to any of [9] to [9d], wherein the intermittent administration is administration for not more than 23 hours per day.
[9f] The adrenomedullin or a derivative thereof having adrenomedullin activity, or a pharmaceutically acceptable salt thereof, or a pharmaceutically acceptable solvate thereof according to any of [9] to [9e], wherein the intermittent administration is administration for not more than 23 hours per day, and is performed for 3 to 30 days.

AM or a derivative thereof having adrenomedullin activity, or a pharmaceutically acceptable salt thereof, or a pharmaceutically acceptable solvate thereof, in the present aspect has characteristics similar to those of the active ingredient of the medicament of the present aspect described above. In addition, AM etc. in the present aspect can be practiced by usage and dosage similar to those of the medicament of the present aspect described above. Furthermore, AM etc. in the present aspect can treat diseases similar to those for the medicament of the present aspect described above.

The present invention also relates to the following.
[10] Use of adrenomedullin or a derivative thereof having adrenomedullin activity, or a pharmaceutically acceptable salt thereof, or a pharmaceutically acceptable solvate thereof, in the manufacture of a therapeutic medicament for a disease,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   wherein the aforementioned therapeutic medicament is intermittently administered.
[10a] The use according to [10], wherein the aforementioned adrenomedullin or the derivative thereof having adrenomedullin activity is a peptide selected from the group consisting of the following:
   (i) a peptide consisting of the amino acid sequence of adrenomedullin,
   (ii) a peptide consisting of the amino acid sequence of adrenomedullin, wherein two cysteine residues in the amino acid sequence form a disulfide bond,
   (iii) the peptide of (ii), wherein the aforementioned disulfide bond is substituted by an ethylene group, and the peptide has adrenomedullin activity,
   (iv) the peptide of any of (i) to (iii), wherein 1 to 15 amino acid residues are deleted, substituted, or added, and the peptide has adrenomedullin activity,
   (v) the peptide of any of (i) to (iv), wherein the C-terminus is amidated, and
   (vi) the peptide of any of (i) to (iv), wherein a glycine residue is added to the C-terminus.
[10b] The use according to [10] or [10a], wherein the aforementioned adrenomedullin or the derivative thereof having adrenomedullin activity is a peptide selected from the group consisting of the following:
   (a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
   (b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
   (c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
   (d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
   (e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
   (f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
   (g) the peptide of any of (a) to (f), wherein the aforementioned disulfide bond is substituted by an ethylene group, and the peptide has adrenomedullin activity;
   (h) the peptide of any of (a) to (g), wherein 1 to 15 amino acid residues are deleted, substituted, or added, and the peptide has adrenomedullin activity;
   (i) the peptide of any of (a) to (h), wherein the C-terminus is amidated; and
   (j) the peptide of any of (a) to (h), wherein a glycine residue is added to the C-terminus.
[10c] The use according to any of [10] to [10b], wherein the aforementioned disease is a disease for which a therapeutic effect is expected by the anti-inflammatory action, angiogenic action, cardiovascular protective action and/or tissue repair-promoting action of adrenomedullin or the derivative thereof having adrenomedullin activity, or the pharmaceutically acceptable salt thereof, or the pharmaceutically acceptable solvate thereof.
[10d] The use according to any of [10] to [10c], wherein the aforementioned disease is at least one selected from the group consisting of cerebrovascular diseases, dementia, inflammatory bowel diseases, autoimmune diseases, infectious diseases, pulmonary hypertension, peripheral vascular diseases, and heart diseases.
[10e] The use according to any of [10] to [10d], wherein the intermittent administration is administration for not more than 23 hours per day.
[10f] The use according to any of [10] to [10e], wherein the intermittent administration is administration for not more than 23 hours per day, and is performed for 3 to 30 days.

AM or a derivative thereof having adrenomedullin activity, or a pharmaceutically acceptable salt thereof, or a pharmaceutically acceptable solvate thereof, used in the present aspect has characteristics similar to those of the active ingredient of the medicament of the present aspect described above. In addition, use of the present aspect can be practiced by usage and dosage similar to those of the medicament of the present aspect described above. Furthermore, in the use of the present aspect, diseases similar to those for the medicament of the present aspect described above can be treated.

Also, in one embodiment of the present invention, the following is provided.
[11] Use of adrenomedullin or a derivative thereof having adrenomedullin activity, or a pharmaceutically acceptable salt thereof, or a pharmaceutically acceptable solvate thereof, for the treatment of a disease,
   wherein the aforementioned disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
   which includes intermittent administration.
[11a] The use according to [11], wherein the aforementioned adrenomedullin or the derivative thereof having adrenomedullin activity is a peptide selected from the group consisting of the following:
   (i) a peptide consisting of the amino acid sequence of adrenomedullin,
   (ii) a peptide consisting of the amino acid sequence of adrenomedullin, wherein two cysteine residues in the amino acid sequence form a disulfide bond,
   (iii) the peptide of (ii), wherein the aforementioned disulfide bond is substituted by an ethylene group, and the peptide has adrenomedullin activity,
   (iv) the peptide of any of (i) to (iii), wherein 1 to 15 amino acid residues are deleted, substituted, or added, and the peptide has adrenomedullin activity,
   (v) the peptide of any of (i) to (iv), wherein the C-terminus is amidated, and
   (vi) the peptide of any of (i) to (iv), wherein a glycine residue is added to the C-terminus.
[11b] The use according to [11] or [11a], wherein the aforementioned adrenomedullin or the derivative thereof having adrenomedullin activity is a peptide selected from the group consisting of the following:
   (a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
   (b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
   (c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
   (d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
   (e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
   (f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
   (g) the peptide of any of (a) to (f), wherein the aforementioned disulfide bond is substituted by an ethylene group, and the peptide has adrenomedullin activity;
   (h) the peptide of any of (a) to (g), wherein 1 to 15 amino acid residues are deleted, substituted, or added, and the peptide has adrenomedullin activity;
   (i) the peptide of any of (a) to (h), wherein the C-terminus is amidated; and
   (j) the peptide of any of (a) to (h), wherein a glycine residue is added to the C-terminus.
[11c] The use according to any of [11] to [11b], wherein the aforementioned disease is a disease for which a therapeutic effect is expected by the anti-inflammatory action, angiogenic action, cardiovascular protective action and/or tissue repair-promoting action of adrenomedullin or the derivative thereof having adrenomedullin activity, or the pharmaceutically acceptable salt thereof, or the pharmaceutically acceptable solvate thereof.
[11d] The use according to any of [11] to [11c], wherein the aforementioned disease is at least one selected from the group consisting of cerebrovascular diseases, dementia, inflammatory bowel diseases, autoimmune diseases, infectious diseases, pulmonary hypertension, peripheral vascular diseases, and heart diseases.
[11e] The use according to any of [11] to [11d], wherein the intermittent administration is administration for not more than 23 hours per day.
[11f] The use according to any of [11] to [11e], wherein the intermittent administration is administration for not more than 23 hours per day, and is performed for 3 to 30 days.

AM or a derivative thereof having adrenomedullin activity, or a pharmaceutically acceptable salt thereof, or a pharmaceutically acceptable solvate thereof, used in the present embodiment has characteristics similar to those of the active ingredient of the medicament of the present aspect described above. In addition, use of the present embodiment can be practiced by usage and dosage similar to those of the medicament of the present aspect described above. Furthermore, in the use of the present embodiment, diseases similar to those for the medicament of the present aspect described above can be treated.

### [Example]

Hereinafter, the present invention is described in more detail using Examples, which does not limit the scope of the present invention. In the present Example, as one example of "a disease for which a therapeutic effect is expected by administration of adrenomedullin or a derivative thereof having adrenomedullin activity, or a pharmaceutically acceptable salt thereof, or a pharmaceutically acceptable solvate thereof", one example is shown in which the present invention was applied to patients with "acute cerebral infarction" which is one of cerebrovascular diseases.

### <Test I: Effect of intermittent administration of adrenomedullin on symptoms of cerebral infarction in patients with acute cerebral infarction>

### [I-1: Test drug]

(a) Active ingredient:
   Synthetic human adrenomedullin (a peptide consisting of the amino acid sequence of SEQ ID NO: 1, having an amidated C-terminus, and in which two cysteine residues in the amino acid sequence form a disulfide bond and form a cyclic structure).
(b) Dosage form, content, and properties:
   An injection agent (freeze-dried product) in which a white mass or powder of 500 µg of synthetic human adrenomedullin is enclosed in one vial. It contains 50 mg of D-mannitol.
(c) Preparation:
   At the time of use, 10 mL of physiological saline is injected into a vial of the test drug with a syringe, and the test drug is dissolved so as not to foam. 10 mL of the dissolved test drug is taken into a syringe, 20 mL of physiological saline is added, and the drug is diluted to a total amount of 30 mL.
(d) Storage:
   After dissolution, it is stored in a sealed container under light-shielding and refrigeration (2-8°C). The control drug (placebo) does not contain an active ingredient (contains only 50 mg of D-mannitol), and is prepared so as to have the same dosage form and properties as the test drug. The test drug and the control drug cannot be distinguished from appearance.

### [I-2: Subjects]

At the National Cerebral and Cardiovascular Center, a National Research and Development Agency, patients with acute cerebral infarction are selected as subjects of the present test. In selection of patients, patients are made subjects who satisfy the selection criteria that administration of a therapeutic drug can be started within 24 hr after onset of cerebral infarction; and that they have neurological signs with an NIHSS score of 1 or more; and who do not conflict with the exclusion criterion that an embolic mechanism is suspected. Patients selected based on the above criteria are assigned to an AM intermittent administration group (20 cases), an AM continuous/intermittent administration group (20 cases), or a placebo group (20 cases).

### [I-3: Administration plan]

### (a) Test drug

### (First-half cohort)

Continuous intravenous administration at 9 ng/kg/min for 8 hr is continued for 7 days. During the drug-free period until 72 hr later, only physiological saline is continuously intravenously administered (AM intermittent administration group).

### (Second-half cohort)

Continuous intravenous administration at 9 ng/kg/min is performed for 72 hr. Thereafter, continuous intravenous administration for 8 hr is continued for 4 days (AM continuous/intermittent administration group).

### (b) Control drug (placebo)

(First-half cohort)
   Same as the test drug.
(Second-half cohort)
   Same as the test drug.

### [I-4: Evaluation items]

(a) Primary evaluation items:
   Safety evaluation item: serious adverse events (SAE) for which a causal relationship cannot be denied
   Efficacy evaluation item: stroke severity evaluation scale (NIHSS)
(b) Secondary evaluation items:
   Safety evaluation item: adverse events (AE)
   Efficacy evaluation item: modified Rankin Scale (mRS)
(c) Other evaluation items:
   Clinical tests (hematological tests and blood biochemical tests are performed)
   Vital signs (blood pressure and electrocardiogram are measured)
   Head image findings (presence or absence of hemorrhage, presence or absence of infarction, and presence or absence of severe stenosis are evaluated by CT and/or MR examination)
   Maximum blood concentration and efficacy evaluation items (d) Exploratory evaluation item:
      AM blood concentration

### [Result-1]

Primary and secondary evaluation items relating to safety and efficacy are shown in Table 1.

**[Table 1]**

| evaluation items | AM intermittent administration group (n=20) | AM continuous/ intermittent administration group (n=20) | placebo group (n=20) | AM intermittent administration group vs. placebo group (adjusted OR [95% CI])** | AM continuous/ intermittent administration group vs. placebo group (adjusted OR [95% CI])** |
|---|---|---|---|---|---|
| primary safety evaluation items | | | | | |
| serious adverse events (SAE) related to intervention, n (%) | 0 (0.0) | 0 (0.0) | 0 (0.0) | - | - |
| secondary safety evaluation items | | | | | |
| all AE, n (%) | 11 (55.0) | 14 (70.0) | 8 (40.0) | - | - |
| primary efficacy evaluation items | | | | | |
| NIHSS score, LSM (95% CI) | | | | | - |
| 8 hr after start of intervention | 2.77 (2.35-3.19) | 3.85 (3.38-4.32) | 3.07 (2.72-3.42) | - | - |
| 24 hr after start of intervention | 2.32 (1.69-2.95) | 3.50 (2.91-4.09) | 2.77 (2.41-3.13) | - | - |
| 72 hr after start of intervention | 2.52 (1.35-3.69) | 3.40 (2.69-4.11) | 2.62 (1.55-3.68) | - | - |
| day 7 | 1.97 (0.92-3.03) | 3.20 (2.46-3.93) | 2.27 (1.23-3.30) | - | - |
| secondary efficacy evaluation items | | | | | |
| mRS score (90 days), n (%)* | | | | | |
| 0-2 (good functional outcome) | 15 (75.0) | 13 (65.0) | 13/19 (68.4) | 1.45 (0.32-6.53) | 0.82 (0.19-3.55) |
| 0-3 (good outcome) | 18 (90.0) | 17 (85.0) | 14/19 (73.7) | 3.70 (0.49-33.33) | 2.86 (0.41-20.00) |

| | | | | | |
|---|---|---|---|---|---|
| * unknown for one patient in placebo group ** adjusted for sex and age | | | | | |

### (Safety evaluation item)

"Serious adverse events (SAE) for which a causal relationship cannot be denied" was made the primary evaluation item, but applicable events were not observed in any of the AM intermittent administration group, the AM
continuous/intermittent administration group, and the placebo group. That is, it was shown that AM intermittent administration in the present invention can be safely performed.

### (Efficacy evaluation item)

The LSM (95% CI) of the NIHSS score in the AM intermittent administration group, the AM continuous/intermittent administration group, and the placebo group were respectively 2.77 (2.35-3.19), 3.85 (3.38-4.32), and 3.07 (2.72-3.42) (8 hr after the start of intervention); 2.32 (1.69-2.95), 3.50 (2.91-4.09), and 2.77 (2.41-3.13) (after 24 hr); 2.52 (1.35-3.69), 3.40 (2.69-4.11), and 2.62 (1.55-3.68) (after 72 hr); and 1.97 (0.92-3.03), 3.20 (2.46-3.93), and 2.27 (1.23-3.30) (final day of intervention). Early neurological improvement of the NIHSS score was observed in 8 cases (40.0%) in the AM intermittent administration group, 3 cases (15.0%) in the AM continuous/intermittent administration group, and 7 cases (35.0%) in the placebo group.

In addition, in 60 cases of non-cardiogenic acute cerebral infarction, outcomes were compared among 20 cases of the AM intermittent administration group, 20 cases of the AM continuous/intermittent administration group, and 20 cases of the placebo group. Patients whose functional outcome was favorable (mRS 0-2) were 15 cases (75.0%) in the AM intermittent administration group, 13 cases (65.0%) in the AM continuous/intermittent administration group, and 13 cases (68.4%) in the placebo group. Furthermore, patients whose functional outcome was ambulatory (mRS 0-3) were 18 cases (90.0%) in the AM intermittent administration group, 17 cases (85.0%) in the AM continuous/intermittent administration group, and 14 cases (73.7%) in the placebo group.

From these results, it was found that the AM intermittent administration group shows a favorable outcome.

### [Result-2]

Taking into consideration the bias in age, sex, and the proportion of intravenous rt-PA therapy in each group, these factors were adjusted, and multivariate analysis was performed for the amount of change in NIHSS. The results are shown in Table 2.

**[Table 2]**

| time | group | N | NIHSS score, LSM (SE) | change in NIHSS from preprocessing, LSM (SE) | difference in NIHSS change between groups, LSM (95% CI) | P value |
|---|---|---|---|---|---|---|
| 8 hr | AM continuous/intermittent administration group | 20 | 3.91 (0.32) | 0.74 (0.32) | 0.86 (0.22-1.49) | 0.01 |
| | AM intermittent administration group | 20 | 3.06 (0.24) | -0.12 (0.24) | - | |
| 24 hr | AM continuous/intermittent administration group | 20 | 3.56 (0.39) | 0.39 (0.39) | 0.96 (0.03-1.88) | 0.04 |
| | AM intermittent administration group | 20 | 2.61 (0.28) | -0.57 (0.28) | - | |
| 72 hr | AM continuous/intermittent administration group | 20 | 3.46 (0.44) | 0.29 (0.44) | 0.66 (-0.79-2.10) | 0.38 |
| | AM intermittent administration group | 20 | 2.81 (0.52) | -0.37 (0.52) | - | |
| 7 days | AM continuous/intermittent administration group | 20 | 3.26 (0.42) | 0.09 (0.42) | 1.01 (-0.35-2.36) | 0.15 |
| | AM intermittent administration group | 20 | 3.26 (0.47) | -0.92 (0.47) | - | |

As is clear from Table 2, the AM intermittent administration group showed statistically significant good results regarding the NIHSS values 8 hr and 24 hr after the start of administration of the investigational drug.

### [Industrial Applicability]

According to the present invention, because the therapeutic effect of AM is high, the present invention is extremely useful in the treatment of a disease for which a therapeutic effect is expected by AM. This application is based on a patent application No. 2024-011993 filed in Japan (filing date: January 30, 2024), the contents of which are incorporated in full herein.

## Claims

1. A medicament for treating a disease, comprising, as an active ingredient, adrenomedullin or a derivative thereof having adrenomedullin activity, or a pharmaceutically acceptable salt thereof, or a pharmaceutically acceptable solvate thereof,
wherein the disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
wherein the active ingredient is intermittently administered.

2. The medicament according to claim 1, wherein the adrenomedullin or the derivative thereof having adrenomedullin activity is a peptide selected from the group consisting of the following:
(i) a peptide consisting of the amino acid sequence of adrenomedullin,
(ii) a peptide consisting of the amino acid sequence of adrenomedullin, wherein two cysteine residues in the amino acid sequence form a disulfide bond,
(iii) the peptide of (ii), wherein the disulfide bond is substituted by an ethylene group, and the peptide has adrenomedullin activity,
(iv) the peptide of any of (i) to (iii), wherein 1 to 15 amino acid residues are deleted, substituted, or added, and the peptide has adrenomedullin activity,
(v) the peptide of any of (i) to (iv), wherein the C-terminus is amidated, and
(vi) the peptide of any of (i) to (iv), wherein a glycine residue is added to the C-terminus.

3. The medicament according to claim 1 or 2, wherein the adrenomedullin or the derivative thereof having adrenomedullin activity is a peptide selected from the group consisting of the following:
(a) a peptide consisting of the amino acid sequence of SEQ ID NO: 1, or a peptide consisting of the amino acid sequence of SEQ ID NO: 1, wherein the cysteine residue at position 16 and the cysteine residue at position 21 form a disulfide bond;
(b) a peptide consisting of the amino acid sequence of SEQ ID NO: 4, or a peptide consisting of the amino acid sequence of SEQ ID NO: 4, wherein the cysteine residue at position 16 and the cysteine residue at position 21 form a disulfide bond;
(c) a peptide consisting of the amino acid sequence of SEQ ID NO: 6, or a peptide consisting of the amino acid sequence of SEQ ID NO: 6, wherein the cysteine residue at position 16 and the cysteine residue at position 21 form a disulfide bond;
(d) a peptide consisting of the amino acid sequence of SEQ ID NO: 8, or a peptide consisting of the amino acid sequence of SEQ ID NO: 8, wherein the cysteine residue at position 16 and the cysteine residue at position 21 form a disulfide bond;
(e) a peptide consisting of the amino acid sequence of SEQ ID NO: 10, or a peptide consisting of the amino acid sequence of SEQ ID NO: 10, wherein the cysteine residue at position 14 and the cysteine residue at position 19 form a disulfide bond;
(f) a peptide consisting of the amino acid sequence of SEQ ID NO: 12, or a peptide consisting of the amino acid sequence of SEQ ID NO: 12, wherein the cysteine residue at position 14 and the cysteine residue at position 19 form a disulfide bond;
(g) the peptide of any of (a) to (f), wherein the disulfide bond is substituted by an ethylene group, and the peptide has adrenomedullin activity;
(h) the peptide of any of (a) to (g), wherein 1 to 15 amino acid residues are deleted, substituted, or added, and the peptide has adrenomedullin activity;
(i) the peptide of any of (a) to (h), wherein the C-terminus is amidated; and
(j) the peptide of any of (a) to (h), wherein a glycine residue is added to the C-terminus.

4. The medicament according to claim 1 or 2, wherein the disease is a disease for which a therapeutic effect is expected by the anti-inflammatory action, angiogenic action, cardiovascular protective action and/or tissue repair-promoting action of adrenomedullin or the derivative thereof having adrenomedullin activity, or the pharmaceutically acceptable salt thereof, or the pharmaceutically acceptable solvate thereof.

5. The medicament according to claim 1 or 2, wherein the disease is at least one selected from the group consisting of cerebrovascular diseases, dementia, inflammatory bowel diseases, autoimmune diseases, infectious diseases, pulmonary hypertension, peripheral vascular diseases, and heart diseases.

6. The medicament according to claim 1 or 2, wherein the intermittent administration is administration for not more than 23 hours per day.

7. The medicament according to claim 1 or 2, wherein the intermittent administration is administration for not more than 23 hours per day, and is performed for 3 to 30 days.

8. A method for treating a disease, comprising administering to a subject a therapeutically effective amount of adrenomedullin or a derivative thereof having adrenomedullin activity, or a pharmaceutically acceptable salt thereof, or a pharmaceutically acceptable solvate thereof,
wherein the disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
wherein the administration is intermittent administration.

9. Adrenomedullin or a derivative thereof having adrenomedullin activity, or a pharmaceutically acceptable salt thereof, or a pharmaceutically acceptable solvate thereof, for use in the treatment of a disease,
wherein the disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
which is intermittently administered.

10. Use of adrenomedullin or a derivative thereof having adrenomedullin activity, or a pharmaceutically acceptable salt thereof, or a pharmaceutically acceptable solvate thereof, in the manufacture of a therapeutic medicament for a disease,
wherein the disease is a disease for which a therapeutic effect is expected by administration of adrenomedullin or a derivative thereof having adrenomedullin activity, or a pharmaceutically acceptable salt thereof, or a pharmaceutically acceptable solvate thereof, and
wherein the therapeutic medicament is intermittently administered.
